# EUROPEAN PATENT APPLICATION

(11) **EP 1 813 617 A1**
(43) Date of publication of application: **01.08.2007**
(21) Application number: 05806279.5
(22) Date of filing: 11.11.2005
(51) Int. Cl.: C07D 491/056, C07D 221/18

(54) **PROCESS FOR PRODUCING BENZOÝc¨PHENANTHRIDINE DERIVATIVE**

(30) Priority: 17.11.2004 JP 2004333763; 17.11.2004 JP 2004333776
(71) Applicant: NIPPON KAYAKU KABUSHIKI KAISHA, Tokyo 102-8172 (JP)
(72) Inventor: YAMAZAKI, Hiroko, Tokyo 114-0024 (JP); MASUDA, Akira, Saitama-shi, Saitama 338-0001 (JP)
(74) Representative: Gille Hrabal Struck Neidlein Prop Roos
(86) International application number: PCT/JP2005/020733
(87) International publication number: WO 2006/054494

(57) **Abstract**

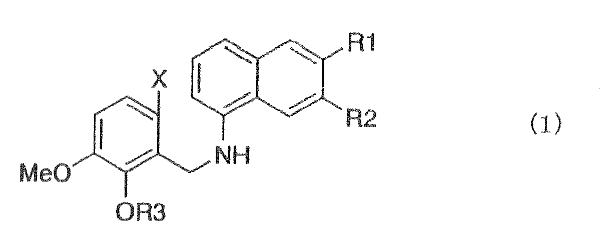

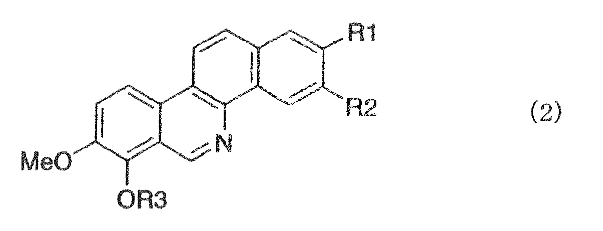

A compound represented by the following formula (1): [wherein R1 and R2 each independently represents hydroxy, provided that R1 and R2 may be bonded to each other to form methylenedioxy, etc.; X represents halogeno; and R3 represents a protective group] is subjected to a cyclization reaction with the aid of an organic silyl hydride and then aromatized with an oxidizing agent to produce a benzo[c]phenanthridine derivative represented by the following formula (2): [wherein R1, R2, and R3 have the same meanings as defined above].

## Description

### TECHNICAL FIELD

The present invention relates to a novel process for producing a benza[c]phenanthridine derivative as an intermediate of a benzo[c]phenanthridinium derivative which has antitumor activity and inhibitory effect on platelet aggregation and is expected as a drug.

### BACKGROUND ART

At present, alkylating agents, nucleic acid antimetabolites, antibiotics, plant alkaloids and the like are used in chemotherapies for cancer patients. It is known that thrombosis is caused by the adhesion and aggregation of platelets and participates not only in cerebral infarction, circulatory organ diseases, carcinomatous DIC and the like but also in cancer metastasis.

Various organic compounds have been proposed as those that are expected to be effective in curing cancer patients and treating thrombosis. For example, patent documents 1 and 2 describe benzo[c]phenanthridinium derivatives having a lower alkyl group as substituent at the 5-position, as having antitumor activity and inhibitory effect on platelet aggregation. Patent document 3 describes benzo[c]phenanthridinium derivatives having a structure formed by the connection of a nitrogen atom at the 5-position to a carbon atom at the 6-position by an aliphatic hydrocarbon chain, as having antitumor activity.
The above-mentioned benzo[c]phenanthridinium derivatives described in patent documents 1 and 2 are synthesized by using as an intermediate a benzo[c]phenanthridine derivative represented by the general formula (2) or general formula (4) shown hereinafter, and patent documents 1 and 2 report that this intermediate is obtained by a ring-closing reaction using an organotin hydride and an aromatization reaction using an oxidizing agent. The benzo[c]phenanthridinium derivatives described in patent document 3 are synthesized by using as an intermediate a benzo[c]phenanthridine derivative represented by the general formula (8) shown hereinafter, and patent document 3 reports that this intermediate is obtained from a 7-benzyloxy-8-methoxybenzo[c]phenanthridine derivative by methylation, the introduction of a lower alcohol, a reaction with an organometallic compound and then an aromatization reaction using an oxidizing agent.

Thus, the production processes of a benzo[c]phenanthridine derivative of the general formula (2) or general formula (4) described in patent documents 1 and 2 use the organotin hydride. The production process of a benzo[c]phenanthridine derivative of the general formula (8) described in patent document 3 requires many steps such as the methylation, lower alcohol introduction, reaction with an organometallic compound, aromatization reaction using an oxidizing agent, and the like for the production from the 7-benzyloxy-8-methoxybenzo[c]phenanthridine derivative.
The production processes of a benzo[c]phenanthridine derivative of the general formula (2) or general formula (4) using the organotin hydride (hereinafter referred to as "stannane reagent") in the reaction involves a serious problem from the viewpoint of the safety of the reaction step, the subsequent after-treatment and the like because the stannane reagent is toxic. The production process of a benzo[c]phenanthridine derivative of the general formula (8) described in patent document 3 is troublesome because it comprises many reaction steps.

Patent document 1: JP-A-5-208959
Patent document 2: JP-A-7-258218
Patent document 3: International Publication No. WO98/23614 pamphlet

### DISCLOSURE OF THE INVENTION

### Problem to be Solved by the Invention

For the production of a benzo[c]phenanthridinium derivative useful as an antitumor agent as described above, there has been a desire for a process for producing an intermediate of such a derivative which is a process not using a stannane reagent or is a simple process comprising a few steps.
Therefore, an object of the present invention is to provide a process for producing a benzo[c]phenanthridine derivative of the general formula (2) or general formula (4) without using a stannane reagent. Another object of the present invention is to provide a simple process for producing a benzo[c]phenanthridine derivative of the general formula (8) which comprises a few steps.

### Means for Solving the Problem

The present inventor earnestly investigated and consequently has found that in a process for producing a benzo[c]phenanthridine derivative of the general formula (2) or general formula (4) as an intermediate of a benzo[c]phenanthridinium derivative which is an antitumor agent, the intermediate is advantageously obtainable by the rapid progress of an organic radical reaction with the aid of an organic silyl hydride without the use of a toxic stannane reagent, and moreover, the present inventor has found a novel simple synthesis route for a benzo[c]phenanthridine derivative of the general formula (8) as an intermediate of a benzo[c]phenanthridinium derivative which comprises a few steps, whereby the present invention has been accomplished.

That is, the present invention is a process for producing a benzo[c]phenanthridine derivative represented by the general formula (2): 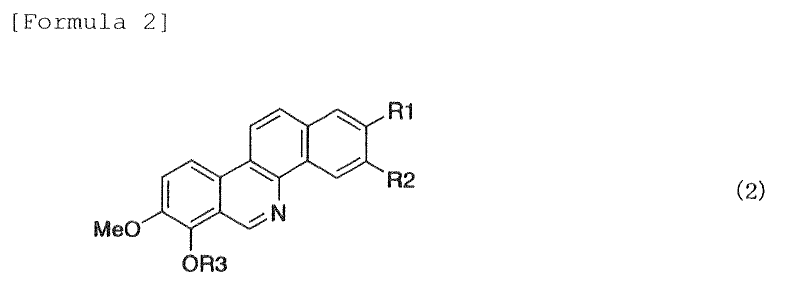 wherein R1, R2 and R3 are as defined below, which is characterized by subjecting a compound represented by the following general formula (1): 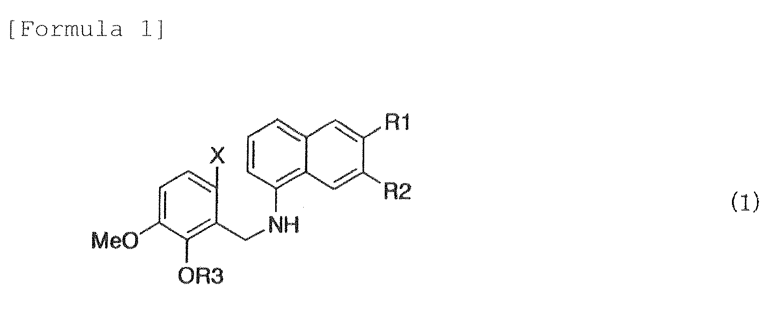 wherein R1 and R2 are independently a hydroxyl group, a hydrogen atom or a lower alkoxy group, or R1 and R2 are bonded to each other to form a methylenedioxy group; X is a halogen atom; and R3 is a protective group, to a ring-closing reaction using an organic silyl hydride, and then aromatizing the reaction product with an oxidizing agent.
More specifically, the present invention is a process for producing a benzo[c]phenanthridine derivative represented by the general formula (4): 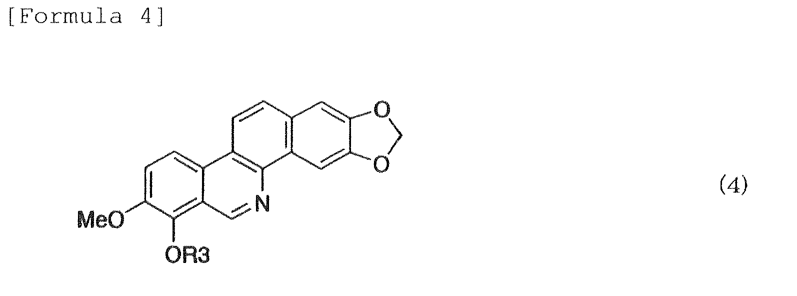 wherein R3 is as defined below, by subjecting a compound represented by the following general formula (3): 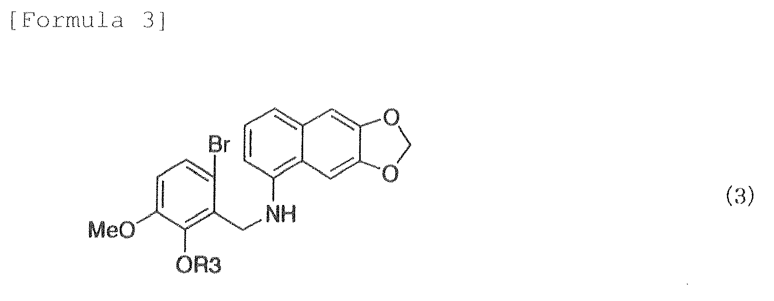 wherein R3 is a protective group, to a ring-closing reaction using an organic silyl hydride, and then aromatizing the reaction product with an oxidizing agent.
In such a production process of the present invention, tris(trimethylsilyl)silane is especially preferable as the organic silyl hydride.

In addition, the present invention is a process for producing a benzo[c]phenanthridine derivative represented by the following general formula (8): 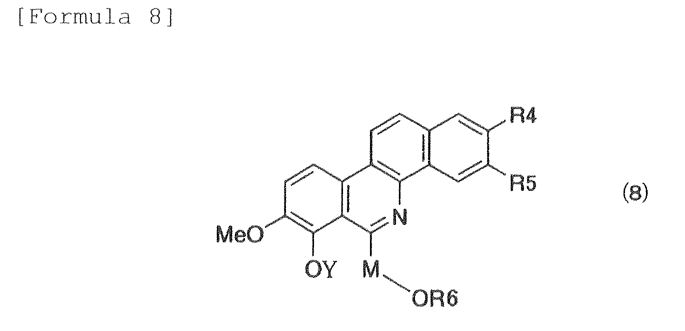 wherein Y is a protective group and R4, R5, R6 and M are as defined below, which is characterized by reacting a compound represented by the following general formula (5): 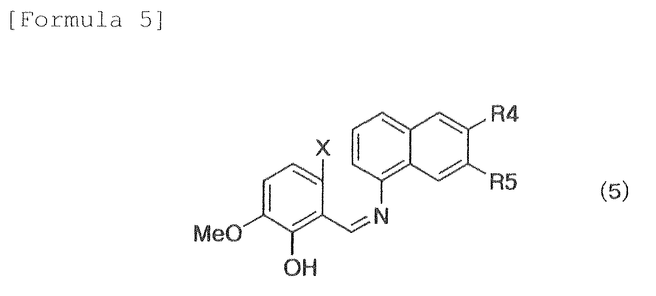 wherein R4 and R5 are independently a hydroxyl group, a hydrogen atom or a lower alkoxy group, or R4 and R5 are bonded to each other to form a methylenedioxy group, and X is a halogen atom, with an organometallic compound represented by the following general formula (6): 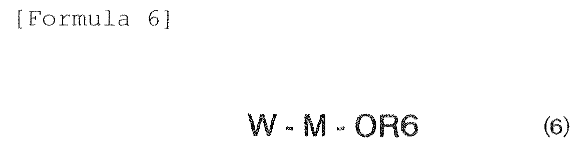 wherein M is an optionally substituted aliphatic hydrocarbon chain; R6 is a protective group; and W is an organic metal or inorganic metal salt, to obtain a compound represented by the following general formula (7): 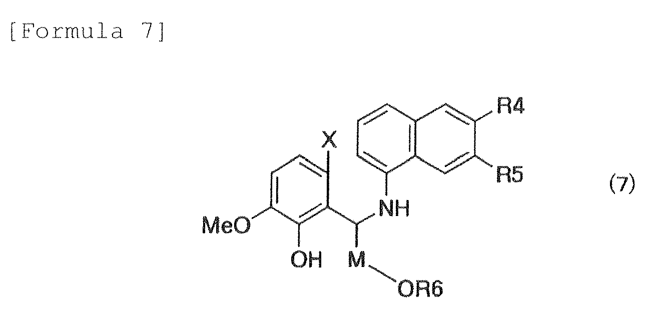 wherein R4, R5, R6, X and M are as defined above, protecting the phenolic hydroxyl group of this compound, subjecting the resulting compound to a ring-closing reaction, and then aromatizing the reaction product with an oxidizing agent.
In the above-mentioned production process of a derivative of the general formula (8), it is preferable to use a compound of the general formula (5) in which R4 and R5 are bonded to each other to form a methylenedioxy group, and a compound of the general formula (6) in which W is an inorganic metal salt and M is a linear aliphatic hydrocarbon chain of 1 to 5 carbon atoms.

### Advantages of the Invention

Owing to the present invention, in the production of a benzo[c]phenanthridine derivative useful as an intermediate of an antitumor agent, an organic radical reaction proceeds rapidly with the aid of a less toxic organic silyl hydride without the use of a toxic stannane reagent, so that it has become possible to produce a desired compound of the general formula (2) or general formula (4) with a simple apparatus under conditions harmless to the environment. Furthermore, the present invention permits efficient production of a benzo[c]phenanthridine derivative substituted at the 6-position and represented by the general formula (8) which is similarly useful as an intermediate of an antitumor agent, by reducing the number of steps for the production.

### BEST MODE FOR CARRYING OUT THE INVENTION

In the present invention, preferable examples of the lower alkoxy group are alkoxy groups of 1 to 5 carbon atoms. Specific examples thereof are methoxy, ethoxy, n-propoxy, isopropoxy, n-butoxy, isobutoxy, t-butoxy, n-pentoxy, etc. Of these, alkoxy groups of 1 to 3 carbon atoms, such as methoxy, ethoxy, n-propoxy and isopropoxy are especially preferable.

In the present invention, preferable examples of lower alkyl group are alkyl groups of 1 to 5 carbon atoms. Specific examples thereof are methyl, ethyl, n-propyl, isopropyl, n-butyl, isobutyl, t-butyl, n-pentyl, etc. Of these, alkyl groups of 1 to 3 carbon atoms, such as methyl, ethyl, n-propyl and isopropyl are especially preferable.

In the present invention, the halogen atom includes fluorine atom, chlorine atom, bromine atom, iodine atom, etc.

In the present invention, the protective group R3 is not particularly limited so long as it is a protective group for phenolic hydroxyl group. The protective group R3 includes, for example, substituted or unsubstituted acyl groups of 2 to 8 carbon atoms, such as acetyl, propionyl, butyryl, isobutyryl, benzoyl, chlorobenzoyl, methylbenzoyl, etc.; branched alkyl or alkylene groups of 3 to 10 carbon atoms, such as isopropyl, isobutyl, t-butyl, isopentyl, 2-butenyl, 3-methyl-2-butenyl, etc.; and substituted or unsubstituted benzyl groups such as benzyl, p-chlorobenzyl, p-trifluorobenzyl, etc. As the substituents of the substituted acyl groups of 2 to 8 carbon atoms and the substituted benzyl groups, there are exemplified lower alkoxy groups, lower alkyl groups, halogen atoms, and lower alkyl groups substituted by a halogen atom(s). Of these, the substituted or unsubstituted benzyl groups and the branched alkyl groups of 3 to 10 carbon atoms (in particular, branched alkyl groups of 3 to 5 carbon atoms) are preferable as the protective group R3.

In the present invention, the protective group R6 is not particularly limited so long as it is a group generally used for protecting a hydroxyl group. The protective group R6 includes, for example, substituted methyl groups such as methoxymethyl, benzyloxymethyl, tetrahydrofuryl, t-butyl, p-methoxybenzyl, triphenylmethyl, etc.; tri(C1-C6)alkylsilyl groups such as t-butyldimethylsilyl, trimethylsilyl, etc.; and substituted or unsubstituted acyl groups of 2 to 8 carbon atoms, such as acetyl, chloroacetyl, benzoyl, isobutyryl, etc. Here, as the substituents of the substituted acyl groups of 2 to 8 carbon atoms, there are exemplified lower alkoxy groups, lower alkyl groups, halogen atoms, and lower alkyl groups substituted by a halogen atom(s). Of these, the tri(C1-C6)alkylsilyl groups, in particular, t-butyldimethylsilyl group are preferable.

As the compound represented by the general formula (1), the following compounds are exemplified. The compound of the present invention is not limited by them:
N-(2'-benzyloxy-6'-bromo-3'-methoxybenzyl)-6,7-methylenedioxy-1-naphthylamine;
N-(2'-benzyloxy-6'-bromo-3'-methoxybenzyl)-6-methoxy-7-isopropoxy-1-naphthylamine;
N-(2'-benzyloxy-6'-bromo-3'-methoxybenzyl)-6-isopropoxy-7-methoxy-1-naphthylamine;
N-(2'-benzyloxy-6'-bromo-3'-methoxybenzyl)-6,7-diisopropoxy-1-naphthylamine; and
N-(2'-benzyloxy-6'-bromo-3'-methoxybenzyl)-1-naphthylamine.
As the compound represented by the general formula (1), compounds represented by the general formula (3) are especially preferable. Of the above-exemplified compounds, N-(2'-benzyloxy-6'-bromo-3'-methoxybenzyl)-6,7-methylenedioxy-1-naphthylamine is preferable.

As the compound represented by the general formula (2), the following compounds are exemplified. The compound of the present invention is not limited by them:
7-benzyloxy-8-methoxy-2,3-methylenedioxybenzo[c]phenanthridine;
7-benzyloxy-3-isopropoxy-2,8-dimethoxybenzo[c]phenanthridine;
7-benzyloxy-2-isopropoxy-3,8-dimethoxybenzo[c]phenanthridine;
7-benzyloxy-2,3-diisopropoxy-8-methoxybenzo[c]phenanthridine; and
7-benzyloxy-8-methoxy-benzo[c]phenanthridine.
As the compound represented by the general formula (2), compounds represented by the general formula (4) are especially preferable. Of the above-exemplified compounds, 7-benzyloxy-2,3-methylenedioxy-8-methoxy-benzo[c]phenanthridine is preferable.

Specific examples of the compound represented by the general formula (5) are the following compounds:
3-bromo-6-methoxy-2-(naphtho[2,3-d][1,3]dioxo-5-yliminomethyl)-phenol;
3-bromo-6-methoxy-2-(naphthalen-1-yliminomethyl)-phenol; and
3-bromo-2-[(6,7-dimethoxynaphthalen-1-ylimino)methyl]-6-methoxyphenol.

In the present invention, M in the general formula (6) represents an optionally substituted aliphatic hydrocarbon chain. As the substituent(s) of the aliphatic hydrocarbon chain, there are exemplified lower alkyl groups, methoxy group, halogen atoms, lower alkoxycarbonyl groups, carbamoyl group, and hydroxyl group protected by a protective group. Here, as the protective group of the hydroxyl group protected by the protective group, there are exemplified the same protective groups as those exemplified above as the protective group R6. As the aliphatic hydrocarbon chain, there are exemplified alkylene groups of 1 to 10 carbon atoms and alkenylene groups of 2 to 10 carbon atoms. Specific examples of the aliphatic hydrocarbon chain for M are methylene, ethylene, n-propylene, isopropylene, 2-methoxyethylene, 2-acetoxyethylene, allylene, 2-butenylene, 3-methyl-2-butenylene, methoxycarbonylmethylene, isopropoxycarbonylmethylene, carbamoylmethylene, etc. In particular, linear aliphatic hydrocarbon chains such as methylene, ethylene and n-propylene are preferable.

In the present invention, W in the general formula (6) represents an organic metal or inorganic metal salt. As the organic metal salt, alkyltins are exemplified. As the inorganic metal salt, there are exemplified salts of lithium, magnesium, aluminum, zinc or copper and a halogen, and magnesium salts are preferable. The organometallic compound represented by the general formula (6) includes organolithium compounds, organomagnesium compounds, organozinc compounds, organocopper compounds, etc. The organomagnesium compounds are preferable.

Specific examples of the compound represented by the general formula (6) are the following compounds:
3-(t-butyldimethylsiloxy)propylmagnesium bromide;
3-(t-butyldimethylsiloxy)-2-methylpropylmagnesium bromide; and
4-(t-butyldimethylsiloxy)butylmagnesium chloride.

The compounds represented by the general formula (1) and the general formula (5) may be synthesized, for example, as follows.
A naphthylamine derivative represented by the following general formula (9): 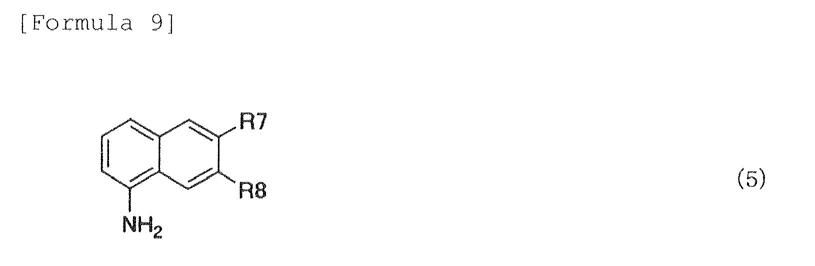 wherein R7 and R8 have the same meanings as those of R1 R2, R4 and R5, and a benzaldehyde derivative represented by the following general formula (10): 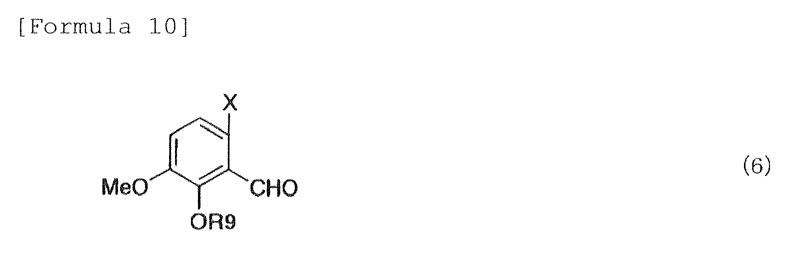 wherein X is a halogen atom and R9 is a hydrogen atom or a protective group, are heated in toluene or benzene at 80°C to 110°C for 1 hour to 3 hours and then concentrated, and the water produced as a by-product by the condensation of the amino group with the aldehyde group of the benzaldehyde derivative is effectively eliminated from the system as an azeotrope with toluene or benzene to effect concentration. If necessary, a procedure of adding fresh toluene or benzene to the concentration residue and concentrating the resulting mixture by heating is repeated two to four times, whereby a dehydrating-condensation product (a Schiff base) may be substantially quantitatively obtained.

The dehydrating-condensation product obtained is the compound represented by the general formula (5) when R9 is a hydrogen atom. When R9 is a protective group, the compound represented by the general formula (1) is obtained by reducing the double bond at the condensation site of the dehydrating-condensation product with a reducing agent. As the reducing agent, any reducing agent may be used so long as it reduces the CN double bond. It is especially preferable to use sodium cyanoborohydride or dimethylaminoborane as the reducing agent and adjust the reaction temperature to a low temperature of -10°C to 40°C.

Next, the production process of the present invention is explained below in detail.

### Process for producing the compound represented by the general formula (2) or general formula (4)

At first, the compound represented by the general formula (1) or general formula (3) is subjected to a ring-closing reaction, i.e., a condensation reaction by the elimination reaction of a hydrogen halide, by the use of an organic silyl hydride preferably in an organic solvent. As the organic silyl hydride, there are exemplified hydrocarbon silyl hydrides having 1 to 10 carbon atoms, preferably hydrocarbon silyl hydrides having 1 to 3 carbon atoms (specific example thereof are tris(trimethylsilyl)silane, triethylsilyl hydride, etc.) and di-hydrocarbon silyl hydrides having 1 to 3 carbon atoms (specific examples thereof are diphenylsilyl hydride, etc). Of these, tris(trimethylsilyl)silane is preferable.
In order to carry out the ring-closing reaction, the compound represented by the general formula (1) or general formula (3) and the organic silyl hydride in an amount of 1 equivalent to 6 equivalent, preferably 1.5 equivalents to 3 equivalents, per equivalent of said compound are dissolved in an organic solvent, preferably a C6-C10 hydrocarbon solvent (e.g. toluene, xylene or benzene), and a free-radical initiator (e.g. 2,2'-azobis(isobutyronitrile), 2,2'-azobis(2-methylbutyronitrile), 2,2'-azobis(2,4'-dimethylvaleronitrile) or benzoyl peroxide) is preferably added thereto, followed by heating at 60°C to 150°C, preferably 80°C to 150°C, for 2 minutes to 4 hours, preferably 5 minutes to 2 hours, whereby the ring closure may be completed.

Then, preferably without separating the product from the reaction mixture, the ring closure portion was oxidatively aromatized with an oxidizing agent at a temperature in the range of 0 to 100°C, preferably 10 to 40°C, for 1 to 120 minutes, preferably 5 to 50 minutes, whereby the compound represented by the general formula (2) or general formula (4) may be obtained. In this reaction various oxidizing agents may be used. For example, active manganese dioxide, lead tetraacetate, mercury acetate or dichlorodicyanobenzoquinone (DDQ), preferably active manganese dioxide is used.
According to need, the compound represented by the general formula (2) or general formula (4), i.e., the desired compound is obtained from the reaction mixture by an isolation and purification method adopted in the case of a conventional organic synthetic reaction.

### Process for producing the compound represented by the general formula (8)

At first, the compound represented by the general formula (5) is dissolved or suspended preferably in an aprotic solvent, for example, an ether solvent such as diethyl ether, diisopropyl ether, 1,2-dimethoxyethane, tetrahydrofuran or the like, followed by adding thereto the organometallic compound represented by the general formula (6) in an amount of 1 to 10 equivalents, preferably 3 to 8 equivalents, per equivalent of the compound represented by the general formula (5), and the resulting mixture is stirred at - 78 to 50°C, preferably 0 to 30°C, for 5 minutes to 24 hours, preferably 10 minutes to 12 hours. Thus, the compound represented by the general formula (7) is obtained .

Specific examples of the compound represented by the general formula (7) are the following compounds:
3-bromo-2-[4-(t-butyldimethylsilanyloxy)-1-(naphtho[2,3-d][1,3]dioxo-5-ylamino)-butyl]-6-methoxyphenol;
3-bromo-2-[4-(t-butyldimethylsilanyloxy)-1-(naphthalen-1-yliminomethyl)-butyl]-6-methoxyphenol; and
3-bromo-2-[4-(t-butyldimethylsilanyloxy)-1-(6,7-dimethoxynaphthalen-1-ylimino)-butyl]-6-methoxyphenol.

As a protective group introduced for protecting the phenolic hydroxyl group of the compound represented by the general formula (7), the same protective groups as those exemplified above as the protective group R3 are used. The protective group may be introduced by a conventional well-known method. For example, the protection with a benzyl group may be carried out, for example, by reacting the compound represented by the general formula (7) with a benzyl halide (e.g. benzyl bromide or benzyl chloride) in dimethylformamide (hereinafter referred to as DMF) in the presence of a base (e.g. potassium carbonate) at 0°C to 50°C, preferably 0°C to 30°C.

Then, the ring-closing reaction may be carried out by the use of a stannane reagent or an organic silyl hydride. Since the stannane reagent is toxic, the organic silyl hydride is preferably used. The ring-closing reaction may be carried out in an organic solvent such as benzene, toluene or xylene. As the organic silyl hydride, there are exemplified hydrocarbon silyl hydrides having 1 to 10 carbon atoms, preferably hydrocarbon silyl hydrides having 1 to 3 carbon atoms (specific examples thereof are tris(trimethylsilyl)silane, triethylsilyl hydride, etc.) and di-hydrocarbon silyl hydrides having 1 to 3 carbon atoms (specific examples thereof are diphenylsilyl hydride). Of these organic silyl hydrides, tris(trimethylsilyl)silane is preferable.

The radical ring-closing reaction using tris(trimethylsilyl)silane is further explained below. The compound obtained by protecting the phenolic hydroxyl group of the compound represented by the general formula (7) and tris(trimethylsilyl)silane in an amount of 1 equivalent to 6 equivalents, preferably 1.5 equivalents to 3 equivalents, per equivalent of the compound obtained are dissolved in an organic solvent, preferably a (C6-C10)hydrocarbon solvent such as toluene, xylene or benzene, and a free-radical initiator such as 2,2'-azobis(isobutyronitrile), 2,2'-azobis(2-methylbutyronitrile), 2,2'-azobis(2,4'-dimethylvaleronitrile) or benzoyl peroxide is preferably added thereto, followed by heating at 60 to 150°C, preferably 80 to 150°C, for 2 minutes to 4 hours, preferably 5 minutes to 2 hours.
A conventional method may be adopted also when an organic silyl hydride other than tris(trimethylsilyl)silane is used.

Although the above-mentioned ring closure product may be isolated, it is preferably not isolated, and the reaction mixture is subjected to aromatization with an oxidizing agent to obtain the compound represented by the general formula (8). The aromatization may be carried out at 0 to 150°C, preferably 10 to 100°C, for 1 to 180 minutes, preferably 5 to 150 minutes.
The oxidizing agent used in this case is not particularly limited. It includes, for example, manganese dioxide, lead tetraacetate, mercury acetate and dichlorodicyanobenzoquinone (DDQ), and active manganese dioxide is preferable.
According to need, the compound represented by the general formula (8), i.e., the desired compound is obtained from the reaction mixture by an isolation and purification method adopted in the case of a conventional organic synthetic reaction.

Specific examples of the compound represented by the general formula (8) are the following compounds:
7-benzyloxy-6-[3-(t-butyldimethylsilanyloxy)propyl]-8-methoxy-2,3-methylenedioxybenzo[c]phenanthridine;
7-benzyloxy-6-[3-(t-butyldimethylsilanyloxy)propyl]-8-methoxy-benzo[c]phenanthridine; and
7-benzyloxy-6-[3-(t-butyldimethylsilanyloxy)propyl]-2,3,8-trimethoxy-benzo[c]phenanthridine.

The compound represented by the general formula (8) may be converted to a compound of the general formula (12), a benzo[c]phenanthridinium derivative having antitumor activity, by the following method according to the same method as described in patent document 3.

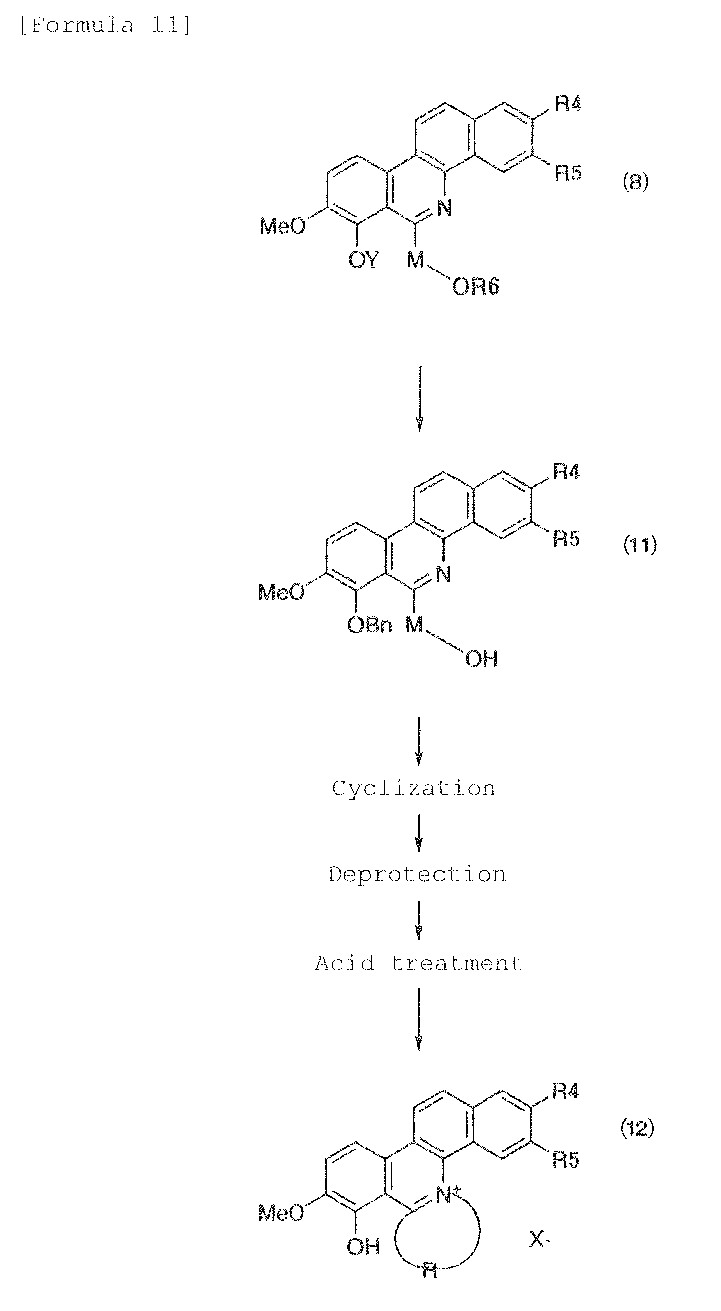

The protective group R6 of the compound represented by the general formula (8) is removed by a method suitable for the protective group. When the protective group R6 is a trialkylsilyl type protective group, the removal is carried out in a solvent such as tetrahydrofuran or acetonitrile by the addition of a fluoride such as tetrabutylammonium fluoride, potassium fluoride or cesium fluoride, at 0 to 80°C, preferably 0°C to room temperature. Also when the protective group R6 is another protective group, it can easily be removed by a well-known deprotection reaction.

A compound represented by the general formula (11) is reacted with an acid chloride (e.g. methanesulfonyl chloride or p-toluenesulfonyl chloride) or an acid anhydride (e.g. trifluoroacetic anhydride) in an organic solvent in the presence of a base such as triethylamine in the temperature range of ice-cooling to room temperature and then the reaction mixture is treated at room temperature to 110°C to carry out cyclization. Thereafter, without isolating and purifying the product, de-benzylation is carried out by treatment under acidic conditions given by concentrated hydrochloric acid or the like, at room temperature to 100°C when the protective group Y is, for example, a benzyl group. Also when the protective group Y is another protective group, it can easily be removed by a well-known deprotection reaction.
Subsequently, the compound obtained by the deprotection is dissolved in a solvent and the resulting solution is subjected to acid treatment by the addition of an acid such as hydrochloric acid, sulfuric acid, methanesulfonic acid or p-toluenesulfonic acid. The amount of the acid is approximately 1 to 3 moles per mole of the compound.
The compound represented by the general formula (12) is obtained by the process described above.

A conventional process for producing the compound represented by the general formula (8) (see patent document 3) is described below.

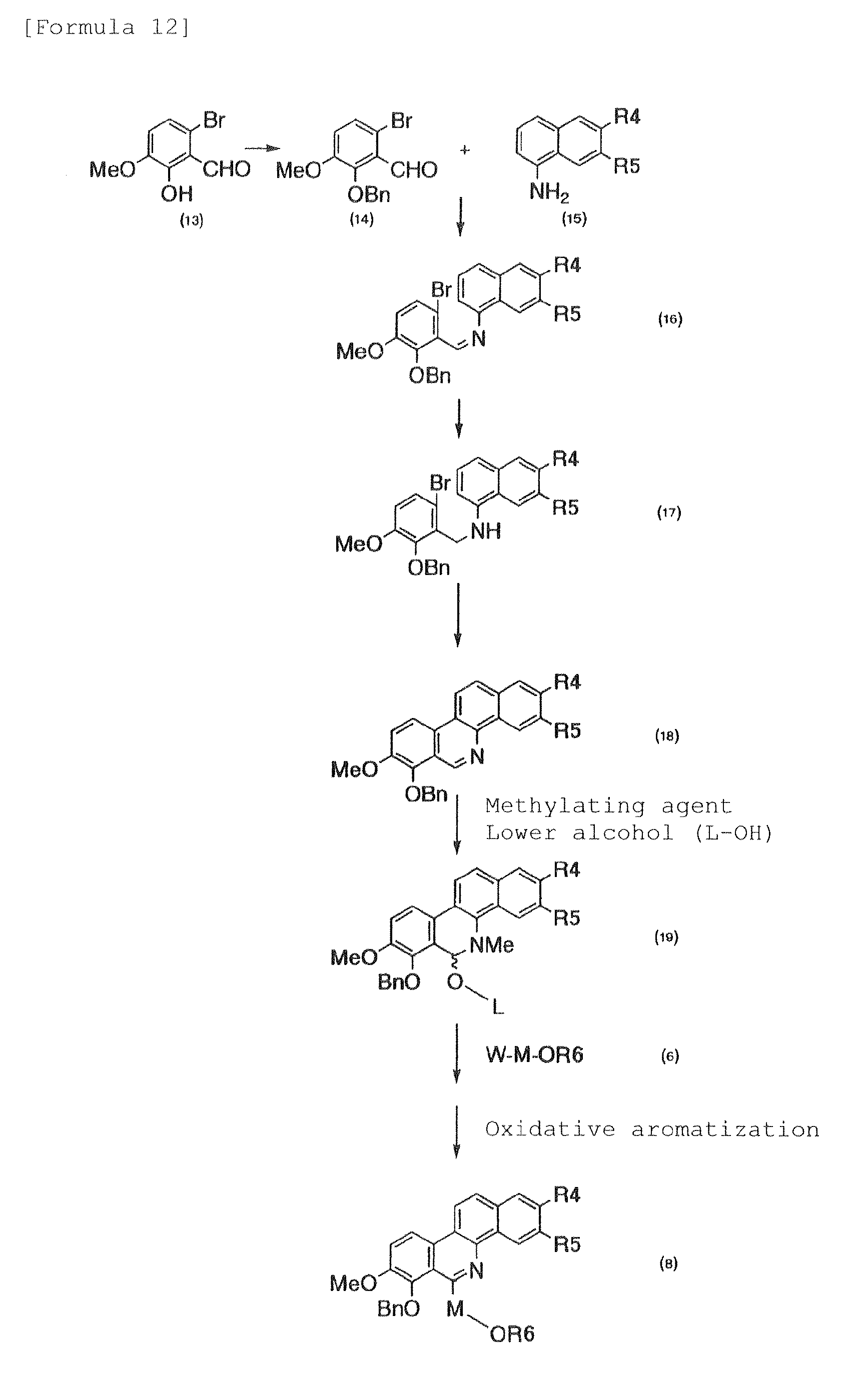

The hydroxyl group of a benzaldehyde derivative of the general formula (13) in which R3 is a hydrogen atom is protected with a benzyl group, and the resulting compound is condensed with a naphthylamine derivative represented by the general formula (15) as described above to obtain a compound represented by the general formula (16). Then, this compound is partially reduced with sodium cyanoborohydride or dimethylaminoboron. Thereafter, the reduced compound is subjected to a radical ring-closing reaction by the use of tributyltin hydride in an organic solvent and then aromatized with an oxidizing agent to obtain a compound represented by the general formula (18).
The compound represented by the general formula (18) is methylated with a methylating agent such as methyl p-toluenesulfonate, methyl 2-nitrobenzenesulfonate or methyl trifluoromethanesulfonate and then mixed with a lower alcohol (L-OH) such as ethanol in the presence of a base to obtain a compound represented by the general formula (19).
The compound represented by the general formula (19) is reacted with an organometallic compound represented by the general formula (6), such as an organomagnesium compound in the presence of an aprotic solvent, and the resulting compound is subjected to oxidative aromatization reaction with an oxidizing agent, whereby the compound represented by the general formula (8) is finally obtained.

On the other hand, in the production process of the present invention, the partial reduction step and methylation step in the above-mentioned conventional production process are omitted. The production process of the present invention realizes the omission of these two steps and makes it possible to obtain the benzo[c]phenanthridine derivative represented by the general formula (8) very easily without deteriorating the reactivity and the yield. This benzo[c]phenanthridine derivative may be converted to a benzo[c]phenanthridinium derivative having antitumor activity by adopting the method described in patent document 3.

Thus, the construction of the benzo[c]phenanthridine skeleton is completed. In the production process of the present invention, the safety of after-treatment and the like is assured without the deterioration of the reactivity and the yield by using an organic silyl hydride in place of the toxic stannane reagent used in the conventional production process. The obtained benzo[c]phenanthridine derivative represented by the general formula (2) or general formula (4) may be converted to a benzo[c]phenanthridinium derivative having antitumor activity by adopting the method described in either of patent documents 1 and 2. In addition, the benzo[c]phenanthridine derivative represented by the general formula (2) corresponds to the above general formula (18) and the benzo[c]phenanthridine derivative represented by the general formula (8) may be obtained therefrom by the methylation, the introduction of a lower alcohol, the reaction with the organometallic compound represented by the general formula (6) and the oxidative aromatization of the ring closure portion with an oxidizing agent which are described in patent document 3.

Examples of the production of the compound according to the present invention are explained below in further detail with reference to working examples, which should not be construed as limiting the scope of the invention.

### Example 1

### Synthesis of 7-benzyloxy-8-methoxy-2,3-(methylenedioxy)-benzo[c]phenanthridine (a compound of the general formula (1) in which R1 and R2 are bonded to each other to form a methylenedioxy group and R3 is a benzyl group, or a compound of the general formula (4) in which R3 is a benzyl group)

In 1L of toluene was dissolved 10 g (20.3 mmol) of N-(2'-benzyloxy-6'-bromo-31-methoxybenzyl)-6,7-methylenedioxy-1-naphthylamine and the resulting solution was refluxed. To this solution were added 7.57 g (30.5 mmol) of tris(trimethylsilyl)silane and 5.85 g (30.5 mmol) of 2,2'-azobis(isobutyronitrile). After 1.5 hours, the reaction mixture was cooled to room temperature and 12 g of active manganese dioxide was added thereto and stirred for 3.5 hours. Then, the manganese was filtered off and the residue was concentrated under reduced pressure. The resulting residue was transferred to a separating funnel together with 300 mL of ethyl acetate and 300 mL of an aqueous sodium hydrogencarbonate solution, followed by extraction with ethyl acetate. The organic layer was dehydrated over anhydrous sodium sulfate, filtered and then concentrated. The residue was dissolved in a small volume of chloroform with heating. After the dissolution was confirmed, 180 mL of hexane was slowly added to the solution and the crystals formed were collected by filtration, washed with hexane and then dried to obtain 4.1 g (9.94 mmol) of the desired compound.

Light-yellow powder
¹H-NMR(200MHz,CDCl₃)ppm:
4.07(s,3H), 5.32(s,2H), 6.13(s,2H), 7.26(s,1H),
7.34~7.46(m,3H), 7.58(dd,J=8.0,1.5Hz,2H),
7.61(d,J=9.1Hz,1H), 7.84(d,J=9.1Hz),
8.34 (d,J=9.0Hz,1H), 8.36 (d, J=8.9Hz, 1H),
8.70(s,1H),9.75(s,1H)

As is clear from Example 1, by the production process of the present invention using an organic silyl hydride, a benzo[c]phenanthridine derivative may be obtained while assuring the safety of after-treatment and the like without deteriorating the reactivity and the yield, without using a toxic stannane reagent used in a conventional production process. The benzo[c]phenanthridine derivative of the general formula (2) or general formula (4) obtained may be converted to a benzo[c]phenanthridinium derivative having antitumor activity by adopting the method described in either of patent documents 1 and 2.

### Example 2

### Synthesis of 7-benzyloxy-6-[3-(t-butyldimethylsilanyloxy)propyl]-8-methoxy-2,3-(methylenedioxy)benzo[c]phenanthridine (a compound of the general formula (8) in which R4 and R5 are bonded to each other to form a methylenedioxy group and R6 is a benzyl group)

### (1) Synthesis of 3-bromo-6-methoxy-2-(naphtho[2,3-d][1,3]dioxo-5-yliminomethyl)-phenol (a compound of the general formula (5) in which R4 and R5 are bonded to each other to form a methylenedioxy group and X is a bromine atom)

1-Naphthylamine (1.67 g, 8.92 mmol) and 2-hydroxy-3-methoxy-6-bromobenzaldehyde (2.06 g, 8.92 mmol) were dissolved in toluene (40 mL) and the resulting solution was heated at 110°C for 3 hours and then concentrated under reduced pressure in a rotary evaporator. To the residue was added 10 mL of fresh toluene and the resulting solution was concentrated under reduced pressure in the same manner as described above. The crystals were collected by filtration and dried to obtain the desired compound as orange-colored powder (2.58 g, 72%).

¹H-NMR(200mHz,C₆D₆)ppm:
9.02(s,1H), 7.68(s,1H), 7.31(d,J=8.1Hz,1H), 7.16(s,1H),
7.02(dd,J=8.1,7.4Hz,1H), 6.92(s,1H),
6.86(d,J=8.5Hz,1H), 6.69(dd,J=7.4,1.1Hz,1H),
6.30(d,J=8.6Hz,1H), 5.20(s,2H),3.37(s,3H)

### (2) Synthesis of 3-bromo-2-[4-(t-butyldimethylsilanyloxy)-1-(naphtho[2,3-d][1,3]dioxo-5-ylamino)-butyl]-6-methoxyphenol (a compound of the general formula (7) in which R4 and R5 are bonded to each other to form a methylenedioxy group; X is a bromine atom; M is a propylene group; and R6 is t-butyldimethylsilanyl)

The 3-bromo-6-methoxy-2-(naphtho[2,3-d] [1,3]dioxo-5-yliminomethyl)-phenol obtained in the above item (1) (10.2 g, 25.5 mmol) was suspended in tetrahydrofuran (THF: 88 mL), followed by adding dropwise thereto 3-(t-butyldimethylsiloxy)propylmagnesium bromide (in an amount corresponding to 186 mmol) over a period of 1 hour. After stirring overnight at room temperature, the reaction mixture was added to a 10% aqueous ammonium chloride solution and extracted with ethyl acetate. The solvent was distilled off from the organic layer under reduced pressure and the residue was washed with hexane to obtain the desired compound as orange-colored powder (11.3 g, 77%).

¹H-NMR(200mHz,CDCl₃)ppm:
7.31(s,1H), 7.15(d,J=8.5Hz,1H), 7.13(d,J=7.0Hz,1H),
7.07(s,1H), 7.07(dd,J=9.5,8.9Hz,1H), 6.68(d,J=7.2,1H),
6.59(d,J=8.7Hz,1H), 6.03(dd,J=2.4,1.1Hz,2H), 5.10(br t,1H), 3.78(s,3H), 3.69(t,2H), 1.4~2.3(m,4H),
0.88(s,9H), 0.04(s,6H)
FAB-MS(positive mode)m/z:573,575[M]⁺

### (3) Synthesis of [1-(benzyloxy-6-bromo-3-methoxyphenyl)-4-(t-butyldimethylsilanyloxy)butyl]-naphtho[2,3-d][1,3]dioxo-5-ylamine (a compound obtained by benzyletherifying the phenolic hydroxyl group of a compound of the general formula (7) in which R4 and R5 are bonded to each other to form a methylenedioxy group; X is a bromine atom; M is a propylene group; and R6 is t-butyldimethylsilanyl)

The 3-bromo-2-[4-(t-butyldimethylsilanyloxy)-1-(naphtho[2,3-d][1,3]dioxo-5-ylamino)-butyl]-6-methoxyphenol obtained in the above item (2) (0.39 g, 0.69 mmol) was dissolved in DMF (5 mL), followed by adding thereto potassium carbonate (0.10 g, 0.76 mmol) and then benzyl bromide (0.12 g, 0.76 mmol), and the resulting mixture was stirred overnight at room temperature. After completion of the reaction, water was added thereto, followed by extraction with ethyl acetate. The organic layer was dried over anhydrous sodium sulfate and distilled under reduced pressure to remove the solvent. The residue was passed through a silica gel column (eluent: hexane : ethyl acetate = 5 : 1 to 1 : 1) and main fractions were collected and then concentrated under reduced pressure to obtain the desired compound as an orange-colored oil (0.44 g, 97%).

¹H-NMR(200mHz,CDCl₃)ppm:
7.43(br s,6H), 6.98~7.31(m,3H), 6.75(brs,1H), 6.70(br d,J=8.7Hz,1H), 6.56(br d,J=7.0Hz,1H),
5.99(dd,J=3.3,1.2Hz,2H), 5.04(br s,2H), 3.79(br s,3H), 3.62(t,3H), 1.4~1.9(m,5H), 0.86(s,9H),0.01(s,6H)
FAB-MS(positive mode)m/z:663,665[M]⁺

(4) Synthesis of 7-benzyloxy-6-[3-(t-butyldimethylsilanyloxy)propyl]-8-methoxy-2,3-(methylenedioxy)benzo[c]phenanthridine (a compound of the general formula (8) in which R4 and R5 are bonded to each other to form a methylenedioxy group and R6 is a benzyl group)

The [1-(benzyloxy-6-bromo-3-methoxyphenyl)-4-(t-butyldimethylsilanyloxy)butyl]-naphtho[2,3-d][1,3]dioxo-5-ylamine obtained in the above item (3) (0.19 g, 0.28 mmol) was dissolved in 5 mL of toluene and the resulting solution was heated at 110°C. To this solution were added tris(trimethylsilyl)silane (0.14 g, 0.57 mmol) and 2,2'-azobis(isobutyronitrile) (0.93 g, 0.48 mmol). After 2 hours, the reaction mixture was cooled to 100 degrees and 400 mg of active manganese dioxide was added thereto and stirred for 2.5 hours. The manganese was filtered off and the residue was concentrated under reduced pressure. The resulting residue was transferred to a separating funnel together with 300 mL of ethyl acetate and 300 mL of an aqueous sodium hydrogencarbonate solution, followed by extraction with ethyl acetate. The organic layer was dehydrated over anhydrous sodium sulfate, filtered and then concentrated. The residue was passed through a silica gel column (eluent: hexane : ethyl acetate = 5 : 1 to 1 : 1) and objective fractions were collected and then concentrated under reduced pressure to obtain a crude product of the desired compound as an ocherous solid (16 mg).

HPLC data
Column: Xterra RP18(5µm)4.6mm(I.D)x150mm(L)
Temperature: 40°C
Eluent: pump A: 0.1% NEt₃ aqueous solution
   pump B: acetonitrile
Bconc: 0min→20min 75%→95%
UV=270nm
retention time: 10.2min

¹H-NMR(200mHz,CDCl₃)ppm:
8.78(br s,1H), 8.45(d,J=9.3Hz,1H), 8.32(d,J=9.1Hz,1H),
7.78(d,J=9.0Hz,1H), 7.60~7.62(m,3H), 7.35~7.47(m,3H),
7.22(s,1H), 6.12(s,2H), 5.20(s,2H),4.05(s,3H),
3.63~3.75(m,4H), 2.18~2.32(m,2H), 0.77(s,9H),0.02(s,6H)
FAB-MS(positive mode)m/z:582[M+H]⁺

### INDUSTRIAL APPLICABILITY

As is clear from Example 1, by the production process of the present invention using an organic silyl hydride, a benzo[c]phenanthridine derivative may be obtained while assuring the safety of after-treatment and the like without deteriorating the reactivity and the yield, without using a toxic stannane reagent used in a conventional production process. The benzo[c]phenanthridine derivative of the general formula (2) or general formula (4) obtained may be converted to a benzo[c]phenanthridinium derivative having antitumor activity by adopting the method described in either of patent documents 1 and 2.

As is clear from Example 2, by the production process of the present invention in which the partial reduction step and methylation step in the above-mentioned conventional production process are omitted, a benzo[c]phenanthridine derivative represented by the general formula (8) can very easily be obtained without deteriorating the reactivity and the yield. This benzo[c]phenanthridine derivative may be converted to a benzo[c]phenanthridinium derivative having antitumor activity by adopting the method described in patent document 3.

## Claims

1. A process for producing a benzo[c]phenanthridine derivative represented by the general formula (2): 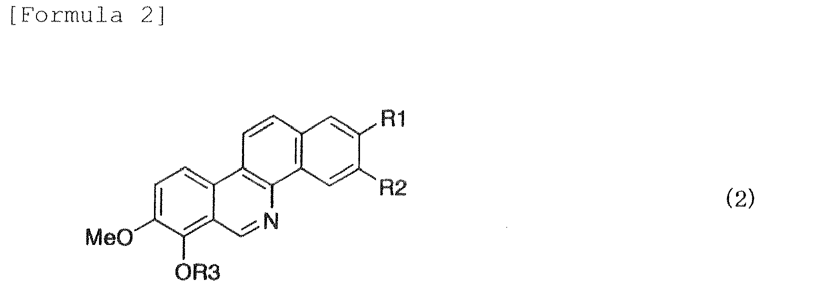 wherein R1, R2 and R3 are as defined below, which is **characterized by** subjecting a compound represented by the following general formula (1): 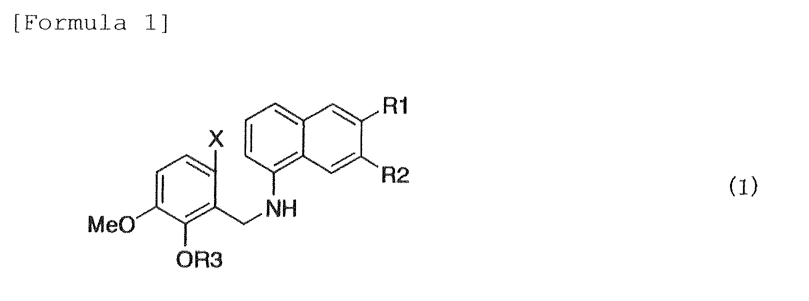 wherein R1 and R2 are independently a hydroxyl group, a hydrogen atom or a lower alkoxy group, or R1 and R2 are bonded to each other to form a methylenedioxy group; X is a halogen atom; and R3 is a protective group, to a ring-closing reaction using an organic silyl hydride, and then aromatizing the reaction product with an oxidizing agent.

2. A production process according to claim 1, wherein a benzo[c]phenanthridine derivative represented by the general formula (4): 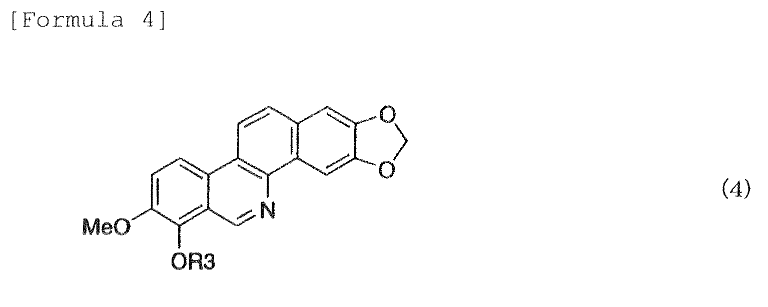 wherein R3 is as defined below, is obtained by subjecting a compound represented by the following general formula (3): 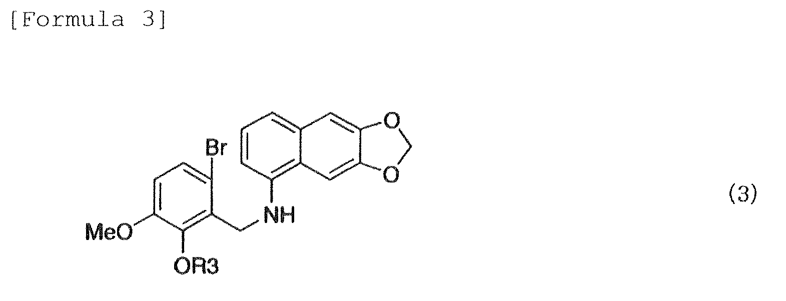 wherein R3 is a protective group, to a ring-closing reaction using an organic silyl hydride, and then aromatizing the reaction product with an oxidizing agent.

3. A process for producing a benzo[c]phenanthridine derivative according to claim 1 or 2, wherein the organic silyl hydride is tris(trimethylsilyl)silane.

4. A process for producing a benzo[c]phenanthridine derivative represented by the following general formula (8): 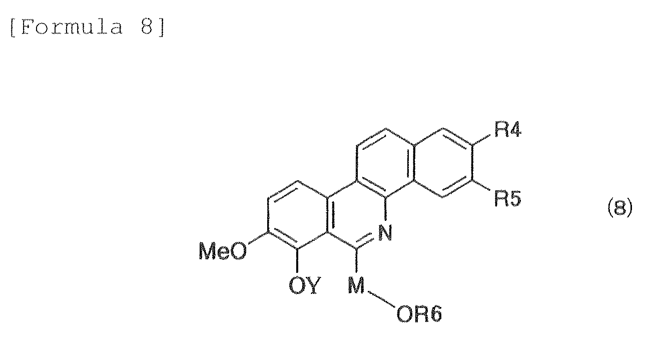 wherein Y is a protective group and R4, R5, R6 and M are as defined below, which is **characterized by** reacting a compound represented by the following general formula (5): 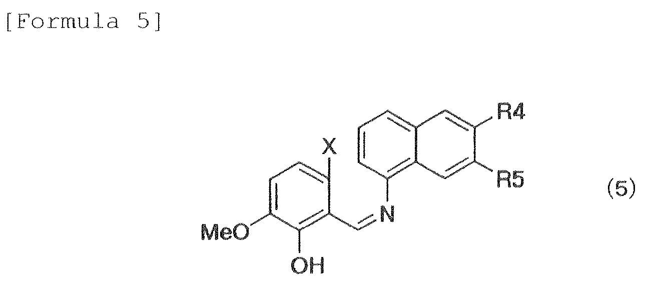 wherein R4 and R5 are independently a hydroxyl group, a hydrogen atom or a lower alkoxy group, or R4 and R5 are bonded to each other to form a methylenedioxy group, and X is a halogen atom, with an organometallic compound represented by the following general formula (6):
[Formula 6] W-M-OR6 (6)
wherein M is an optionally substituted aliphatic hydrocarbon chain; R6 is a protective group; and W is an organic metal or inorganic metal salt, to obtain a compound represented by the following general formula (7): 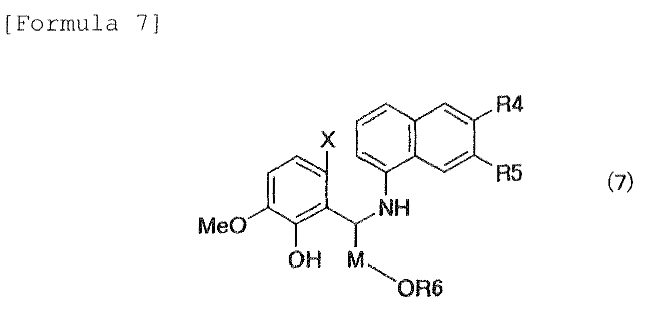 wherein R4, R5, R6, X and M are as defined above, protecting the phenolic hydroxyl group of this compound, subjecting the resulting compound to a ring-closing reaction, and then aromatizing the reaction product with an oxidizing agent.

5. A process for producing a benzo[c]phenanthridine derivative according to claim 4, wherein in the general formula (5), R4 and R5 are bonded to each other to form a methylenedioxy group, and in the general formula (6), W is an inorganic metal salt and M is a linear aliphatic hydrocarbon chain of 1 to 5 carbon atoms.
